(19) Europäisches Patentamt
European Patent Office
Office européen des brevets

(11) Numéro de publication : 0 382 641 B1

# (12) FASCICULE DE BREVET EUROPEEN

(45) Date de publication du fascicule du brevet : **06.07.94 Bulletin 94/27**

(51) Int. Cl.$^5$ : **C07C 69/96,** C07C 68/06

(21) Numéro de dépôt : **90400344.9**

(22) Date de dépôt : **08.02.90**

## (54) Procédé de préparation de dicarbonate de ditertioalcoyle.

(30) Priorité : **10.02.89 FR 8901733**

(43) Date de publication de la demande : **16.08.90 Bulletin 90/33**

(45) Mention de la délivrance du brevet : **06.07.94 Bulletin 94/27**

(84) Etats contractants désignés : **AT BE CH DE ES FR GB GR IT LI LU NL SE**

(56) Documents cités :
**US-A- 3 078 294**
**CHEMICAL ABSTRACTS, vol. 91, no. 7, 13 août 1979, page 649, résumé no. 56376y, Columbus, Ohio, US; N. N. PODGORNOVA et al, "Di-tert-butyl pyrocarbonate"; & SU-A-659 561**

(56) Documents cités :
**CHEMICAL ABSTRACTS, vol. 90, no. 21, 21 mai 1979, page 567, résumé no. 168196a, Columbus, Ohio, US; V. F. POZDNEV et al: "Synthesis of di-tert-butylpyrocarbonate from sodium tert-butylcarbonate and carboxylic acid chlorides"**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière,**
**Route de Ternay,**
**Communay**
**F-69360 St-Symphorien d'Ozon (FR)**

(74) Mandataire : **Ricalens, François et al**
**RHONE-POULENC CHIMIE**
**Service Brevets Chimie**
**25, quai Paul Doumer**
**F-92408 Courbevoie (FR)**

EP 0 382 641 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau procédé de préparation du dicarbonate de ditertioalcoyle. Elle concerne plus particulièrement la préparation du dicarbonate de ditertiobutyle à partir d'halogénure d'acide.

Il est connu depuis longtemps de préparer le dicarbonate de ditertiobutyle encore souvent dénommé $BOC_2O$ à partir du carbonate de tertiobutyle et d'alcalin et de phosgène tel que par exemple décrit par J.H. HOWE dans le journal of organic chemistry 27, 1901 (1962).

L'emploi de phosgène pose de tels problèmes de sécurité que l'industrie cherche à éviter son emploi.

Il est aussi connu selon l'article publié par Pozdev, Smirnova Podgornova, Zentsova et le Kalei dans Zhurnal Urganicheskoï Khimii Vol. 15, n° 1, pages 106-109 en 1979 et traduit dans le Journal of Organic Chemistry USSR 1979, page 95 de préparer le dicarbonate de tertiobutyle par condensation du carbonate de tertiobutyle avec un chlorure d'acide dans un mélange de toluène et de diméthylformamide. Les rendements obtenus, lorsque nous reproduisons ces essais, en dicarbonate de tertiobutyle ne sont pas suffisants.

La présente invention a permis de préparer le dicarbonate de ditertiobutyle en l'absence de phosgène et avec de bons rendements.

Elle consiste à condenser le carbonate de tertiobutyle et d'alcalin avec un halogénure d'acide en présence d'un agent complexant choisi parmi 3 classes d'agents complexants comprenant les amines tertiaires oxygénées, et les polyéthers oxygénés, soufrés, cycliques ou macrocycliques.

La réaction peut être schématisée de la façon suivante :

$$2(CH_3)_3C\text{-}O\text{-}CO_2M + RCOX \Rightarrow (CH_3)_3C\text{-}O\text{-}CO\text{-}O\text{-}CO\text{-}O\text{-}C(CH_3)_3 + R\ CO2\ M + MX$$

dans laquelle X représente un halogène et M un métal alcalin.

Le carbonate de tertiobutyle et d'alcalin est obtenu de manière connue par exemple par carbonatation de tertiobutylate.

Parmi les carbonates de tertiobutyle et d'alcalin, on préfère utiliser les sels alcalins et tout particulièrement le sel de sodium (M=Na).

Les halogénures d'acide utilisables dans le procédé de l'invention sont choisis parmi les chlorures ou bromures d'acides carboxyliques ayant 1 à 7 atomes de carbone éventuellement substitués par des groupements électroattracteurs tels que des atomes d'halogène, des groupements nitro.

On préfère tout particulièrement utiliser les chlorures d'acide carboxyliques ayant 2 atomes de carbone et encore plus préférentiellement le chlorure de trichloroacétyle.

Les agents séquestrants utilisables sont choisis parmi 3 classes d'agents complexants comprenant les amines tertiaires oxygénées, et les polyéthers oxygénés, soufrés, cycliques ou macrocycliques.

La première classe est constituée des agents séquestrants de formule générale.

N - CHR1 - CHR2 - O - (CHR3 - CHR4 - O)n - R5 3 (I)

dans laquelle n est un nombre entier supérieur ou égal à 0 et inférieur ou égal à 10 environ (O < n < 10), R1, R2, R3, R4 identiques ou différents représentent un atome d'hydrogène ou un radical alcoyle ayant de 1 à 4 atomes de carbone et R5 représente un radical alkyle ou cycloalkyle ayant de 1 à 12 atomes de carbone, un radical phényle ou un radical de formule -CmH2m-ø, ou CmH2m+1-ø, m étant compris entre 1 et environ 12.

La deuxième classe d'agents complexants est constituée des polyéthers cycliques de préférence macrocycliques ayant de 6 à 30 atomes dans le cycle et de préférence de 15 à 30 atomes dans le cycle et constitués de 2 à 10 de préférence de 4 à 10 unités -O-X dans lesquelles X est soit -CHR6-CHR7- soit -CHR6-CHR8-CR9R7, R6, R7, R8 et R9 identiques ou différents étant un atome d'hydrogène ou un radical alkyle ayant de 1 à 4 atomes de carbone, un des X pouvant être -CHR6-CHR8CR9R7- quand les unités -O-X comprennent le groupement -O-CHR6-CHR7.

La troisième classe d'agents complexants est constituée des composés de formule générale :

$R_{10}$-Z [A - D]$_p$ A - Z - $R_{10}$ IIa

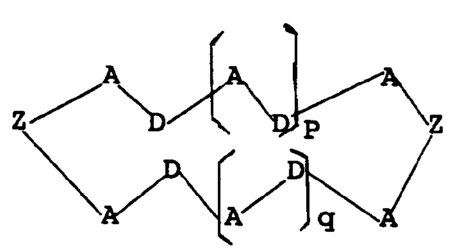

IIb

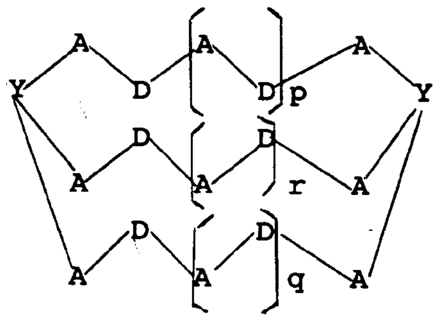

IIc

dans lesquelles :

- Z représente O ou Y-$R_{10}$
- Y représente N ou P
- A représente un groupement alkylène ayant de 1 à 3 atomes de carbone.
- D représente O, S ou N-$R_{11}$ où $R_{11}$ représente un radical alkyle ayant de 1 à 6 atomes de carbone.
- $R_{10}$ peut représenter un radical alkyle ayant 1 à 6 atomes de carbone, p, q et r identiques ou différents sont des nombres entiers compris entre 1 et 5.

Selon un mode de réalisations préférentiel du procédé de l'invention on utilise au moins un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 6, et pour lesquels $R_5$ représente un radical alkyle ayant de 1 à 4 atomes de carbone.

On peut citer :

- la tris(oxa-3 butyl)amine de formule :
  N-(CH2-CH2-O-CH3)3
- la tris(oxa-3 heptyl)amine de formule :
  N-(CH2-CH2-O-C4H9)3
- la tris(dioxa-3,6 heptyl)amine de formule :
  N-(CH2-CH2-O-CH2-CH2-O-CH3)3
- la tris(trioxa-3,6,9 décyl)amine de formule :
  N-(CH2-CH2-O-CH2-CH2-O-CH2-CH2-O-CH3)3
- la tris(dioxa-3,6 octyl)amine de formule :
  N-(CH2-CH2-O-CH2-CH2-O-C2H5)3
- la tris(trioxa-3,6,9 undécyl)amine de formule :
  N-(CH2-CH2-O-CH2-CH2-O-CH2-CH2-O-C2H5)3
- la tris(dioxa-3,6 nonyl)amine de formule :
  N-(CH2-CH2-O-CH2-CH2-O-C3H7)3
- la tris(trioxa-3,6,9 dodécyl)amine de formule :
  N-(CH2-CH2-O-CH2-CH2-O-CH2-CH2-O-C3H7)3
- la tris(dioxa-3,6 décyl)amine de formule :
  N-(CH2-CH2-O-CH2-CH2-O-C4H9)3
- la tris(trioxa-3,6,9 tridécyl)amine de formule :
  N-(CH2-CH2-O-CH2-CH2-O-CH2-CH2-O-C4H9)3
- la tris(tétraoxa-3,6,9,12 tridécyl)amine de formule :
  N-(CH2-CH2-O-(CH2-CH2-O-)3-CH3)3
- la tris(dioxa-3,6 méthyl-4 heptyl)amine de formule :
  N-(CH2-CH2-O-CHCH3-CH2-O-CH3)3
- la tris(dioxa-3,6 diméthyl-2,4 heptyl)amine de formule :
  N-(CH2-CHCH3-O-CHCH3-CH2-O-CH3)3

On préfère utiliser parmi les composés de formule (I) : la tris(dioxaheptyl)amine ci après désignée par le sigle TDA.

La préparation de ces agents séquestrants est décrite dans la demande de brevet français 79 05438 publiée sous le n°2450120.

Les éthers cycliques qui peuvent être mis en oeuvre dans le procédé selon l'invention comprennent des composés comme le dioxanne ou des éthers macrocycliques connus sous l'appellation générale d'"éthers couronnes" qui sont décrits dans le brevet français 69.43879 publié sous le numéro 2.026.481.

On peut citer comme exemples d'éthers couronnes pouvant être utilisés selon l'invention :

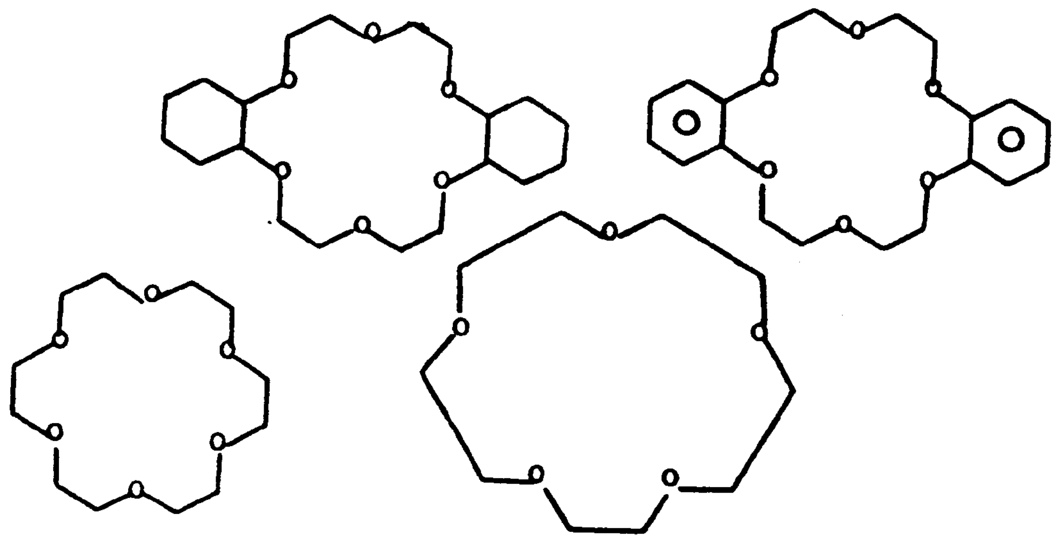

Une partie des composés de la troisième classe d'agents complexants sont décrits dans le brevet français 70.21079 publié sous le numéro 2.052.947. On peut citer comme exemples de composés de cette classe convenant pour la mise en oeuvre du procédé selon l'invention :

$$CH_3 - O - CH_2 - CH_2 - O - CH_2 - CH_2 - OCH_3$$

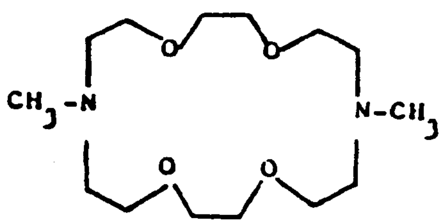

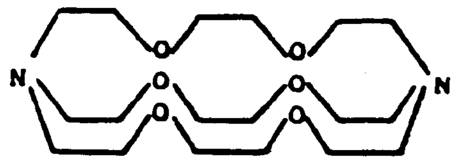

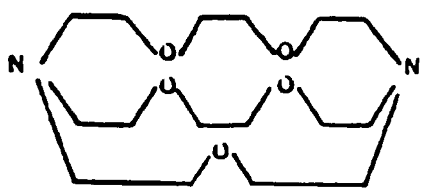

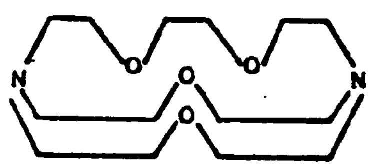

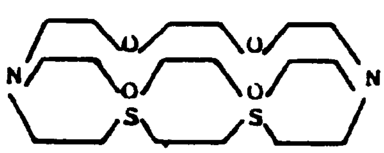

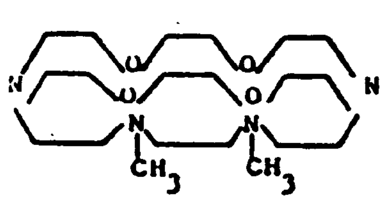

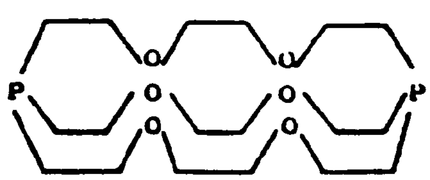

Selon un premier procédé préférentiel de mise en oeuvre de l'invention, on met en contact le tertiobutylate de sodium avec du dioxyde de carbone, on ajoute ensuite le chlorure de trichloracétyle puis on ajoute une quantité supplémentaire de tertiobutylate.

L'agent complexant peut être introduit à tout moment, lors de la carbonatation ou préalablement à l'addition de la quantité supplémentaire de tertiobutylate.

Selon un deuxième procédé préférentiel, la totalité du tertiobutylate est introduit au départ de la réaction, puis on ajoute le chlorure d'acide. Le complexant étant ajouté à tout moment.

Selon un autre procédé de mise en oeuvre, le tertiobutylate est introduit en totalité sur le chlorure d'acide.

La réaction peut être réalisée dans tout solvant quelle que soit la nucléophilie du milieu. On peut citer parmi les solvants :

- les dérivés hydrocarbonées aromatiques tels que :
  le benzène
  les xylènes
  le toluène
- les solvants aprotiques polaires tels que :
  le diméthylformamide
- les solvants oxygénés tels que :
  le dioxanne
  les diéthylène ou diméthylène glycols

D'une manière générale, les meilleurs solvants sont des solvants présentant les propriétés suivantes :

- $F \leqq 10°$ C
- $E_b \leqq 150°$ C
- inerte vis à vis du réactif
- non miscibles à l'eau.

Il est en outre avantageux que les coefficients de partage du complexant et du $BOC_2$ O entre la phase organique et la phase aqueuse soient telle que le complexant passe au moins cent fois mieux dans l'eau que le $BOC_2$ O.

Parmi les solvants préférés répondant aux contraintes ci-dessus, il convient de mentionner les solvants aromatiques et les fractions pétrolières contenant au moins 50 % d'aromatique.

Selon un mode préféré de réalisation de l'invention on utilise une quantité molaire d'halogénure d'acide comprise entre 0,3 et 0,49 fois la quantité de tertiobutylate utilisée.

La température de réaction est avantageusement comprise entre -10°C et 60°C.

La présente invention sera plus complètement décrite à l'aide des exemples suivants qui ne doivent pas être considérés comme limitatifs de l'invention.

1. Mode opératoire type . Influence de la quantité de $TDA_1$

Dans un réacteur de 250 ml, on charge
  12,8 g de tBu ONa (0,133 mole)
  75 ml de toluène sec

A température ambiante, introduit du $CO_2$ ce qui provoque une exothermie. L'introduction est effectuée en trente minutes. L'introduction du $CO_2$ est alors arrêtée et on refroidit le mélange réactionnel à 10°C, température à laquelle on ajoute dans un premier temps x ml de TDA, puis 9,7 g de chlorure de trichloroacétyle (0,0506 mole) dissous dans 30 ml de toluène à 10°C en 10 minutes. Le mélange réactionnel est très épais. On laisse ensuite remonter à température ambiante puis on agite 5 heures 30.

Après refroidissement, on ajoute 100 ml d'eau glacée. La phase organique lavée avec 3 x 100 ml d'eau, séchée sur 15 g de sulfate de sodium est evaporée à température ambiante.

Le produit obtenu de couleur orangée est analysé par CPG.

| Essai | Toluène | Complexant | Température | Durée | RR |
|---|---|---|---|---|---|
| Comparatif | 75 + 30 | - | 25°C | 5h30 | 6 |
| 1 | 75 + 30 | 1 ml TDA 1 (0,003 mole) | 25°C | 5h30 | 53 |
| 2 | 75 + 30 | 2 ml TDA 1 | 25°C | 5h30 | 62 |
| 3 | 75 + 30 | 3 ml TDA 1 | 25°C | 5h30 | 78 |
| 4 | 75 + 30 | 5 ml TDA 1 | 25°C | 5h30 | 77,5 |

On entend par RR le rendement sur le produit introduit c'est-à-dire :

$$\frac{\text{quantité de produit obtenu (moles)}}{\text{quantité de produit attendu (moles)}}$$

2. Influence de la température

On opère comme dans l'exemple 1 en chargeant

0,13 mole de tertiobutylate de sodium

0,051 mole de chlorure de trichloracétyle (CTCA)

75 + 30 ml de toluène

5 ml de TDA 1 (0,0015 mole)

La carbonatation est effectuée à 10°, puis après coulée du CTCA, la température de réaction est ramenée à la valeur T mentionnée dans le tableau. La réaction dure 5h30.

| Essai | Toluène | Complexant | Température T°C | Durée | RR |
|---|---|---|---|---|---|
| 5 | 75 + 30 | 5 ml TDA 1 (0,015 mole) | 10°C | 5h30 | 79 |
| 6 | 75 + 30 | 5 ml TDA 1 | 20°C | 5h30 | 77,5 |
| 7 | 75 + 30 | 5 ml TDA 1 | 28°C | 5h30 | 76 |
| 8 | 75 + 30 | 5 ml TDA 1 | 50°C | 5h30 | 77 |

3 . Influence du rapport tBuONa/CTCA

On opère comme dans l'exemple 1 en chargeant

0,13 mole de tertiobutylate de sodium (TBuONa)

x mole de chlorure de trichloracétyle (CTCA)

75 + 30 ml de toluène

0,015 mole de TDA 1

La réaction est effectuée à 20° pendant 5h30.

| Essai | Toluène | Mole CTCA | Excès TBuONa par rapport à la stoechiométrie | Température | RR |
|---|---|---|---|---|---|
| 9 | 75 + 30 | 0,065 | 0 | 20° | 52,5 |
| 10 | 75 + 30 | 0,062 | 4 % | 20° | 79,5 |
| 11 | 75 + 30 | 0,059 | 9 % | 20° | 80,5 |
| 12 | 75 + 30 | 0,051 | 21 % | 20° | 77,5 |

4 . Influence de la dilution

On opère comme dans l'exemple 1 en chargeant.

0,13 mole de tertiobutylate de sodium
0,051 mole de chlorure de trichloracétyle
x + 30 ml de toluène
0,015 mole de TDA 1

La réaction est effectuée à 20° pendant 5h30

| Essai | Toluène | Température | RR |
|---|---|---|---|
| 13 | 35 + 30 | 20° | 37 |
| 14 | 75 + 30 | 20° | 77,5 |
| 15 | 180 + 30 | 20° | 82 |
| 16 | 290 + 30 | 20° | 77 |

5 . Influence de la durée

On opère comme dans l'exemple 1.

Dans les essais comparatifs, on charge,

0,13 mole de tertiobutylate de sodium
10 ml de DMF (en fin de carbonatation)

0,051 mole de chlorure de trichloracétyle
75 + 30 ml de toluène.

La réaction est effectuée à 20° pendant un temps variable.

| Essai | Toluène | Complexant Solvant | Température | Durée | RR |
|---|---|---|---|---|---|
| Comparatif avec publication de POZDEV utilisant toluène + DMF | 75 + 30 | DMF 10 ml | 10°C | 1h | 9% |
| Comparatif avec publication de POZDEV utilisant toluène + DMF | 75 + 30 | DMF 10 ml | 20°C | 3h30 | 42% |
| Comparatif avec publication de POZDEV utilisant toluène + DMF | 75 + 30 | DMF 10 ml | 20°C | 5h30 | 51% |
| Comparatif avec publication de POZDEV utilisant toluène + DMF | 75 + 30 | DMF 10 ml | 20°C | 16h | 57,3% |

Les essais sont réalisés avec :

0,13 mole de tertiobutylate de sodium
0,051 mole de chlorure de trichloracétyle
75 + 30 ml de toluène
0,015 mole de TDA 1

Température, 20°.

| Essai | Toluène | Complexant | Température | Durée | RR |
|---|---|---|---|---|---|
| 17 | 75 + 30 | 5 ml TDA 1 | 20°C | 1h | 76 |
| 18 | 75 + 30 | 5 ml TDA 1 | 20°C | 1h45 | 82 |
| 19 | 75 + 30 | 5 ml TDA 1 | 20°C | 5h30 | 77,5 |
| 20 | 75 + 30 | 5 ml TDA 1 | 20°C | 22h30 | 75 |

6. Influence du solvant

On opère comme dans l'exemple 1 en chargeant.

0,13 mole de tBuONa
0,051 mole de CTCA
éventuellement,
0,015 mole de TDA 1
75 + 30 ml d'un solvant.

| Essai | Solvant | Complexant | Température | Durée | RR |
|---|---|---|---|---|---|
| Comparatif | Toluène (75 + 30) | | 20° | 5h30 | 6% |
| 4 | Toluène (75 + 30) | 5 ml TDA 1 | 20° | 5h30 | 77,5 |
| 21 | Dioxanne (75 + 30) | | 20° | 5h30 | 23 |
| 22 | Dioxanne (75 + 30) | 5 ml TDA 1 | 20° | 5h30 | 69% |
| 23 | Diglyme (75 + 30) | | 20° | 5h30 | 40% |

7 . Influence de complexant

On opère comme dans l'exemple 1 avec :

0,13 mole de tBuONa
x mole d'un complexant
0,051 mole de CTCA
75 + 30 ml de toluène

| Essai | Toluène | Complexant | Température | Durée | RR |
|---|---|---|---|---|---|
| Comparatif | 75 + 30 | | 20°C | 5h30 | 6% |
| 24 | 75 + 30 | Diglyme (0,13 mole) | 20°C | 5h30 | 43% |
| 25 | 75 + 30 | Dioxanne (0,23 mole) | 20°C | 5h30 | 14% |

8. Influence de l'halogénure d'acide

On opère comme dans l'exemple 1 avec :
0,13 mole de TBuONa
O,051 mole de chlorure d'acide
75 + 30 ml de toluène
0,015 mole de TDA
Température : 20°C
Durée : 5 H 30

| Essai | Toluène | Chlorure d'acide | Température | Durée | RR |
|---|---|---|---|---|---|
| 26 | 75 + 30 | $CCl_3COCl$ | 20°C | 5h30 | 77,5 |
| 27 | 75 + 30 | $CHCl_2COCl$ | 20°C | 5h30 | 20% |
| 28 | 75 + 30 | $CH_2ClCOCl$ | 20°C | 5h30 | 5% |

8. CHANGEMENT DE SUBSTRAT

PYROCARBONATE DE DI TERTIOAMYLE

Dans un réacteur de 250 ml, on introduit 8,1 g de t-amylate de sodium (0,064 mole) 100 ml de toluène, 5 g de $TDA_1$
A cette suspension, on injecte pendant 30 minutes du $CO_2$, jusqu'à ce que le débit en sortie de réacteur soit identique au débit d'entrée. Après avoir refroidi à 0° C, on coule en 10 minutes 3,8 g de chlorure de dichloroacétyle (0,0258 mole). Le mélange réactionnel est ensuite réchauffé à 20°C où il est agité pendant cinq heures. Après lavage, la phase organique est séchée et évaporée. On recueille 1,7 g d'un produit aqueux dans lequel on dose par RMN 88 % de pyrocarbonate de ditertioamyle ce qui correspond à un RR de 23,5 % par rapport au chlorure de dichloroacétyle engagé.

## Revendications

1. Procédé de préparation du dicarbonate de ditertioalcoyle caractérisé en ce que l'on met en contact un carbonate de tertioalcoyle et d'alcalin et un halogénure d'acide en présence d'un agent complexant choisi

parmi les amines tertiaires oxygénées, et les polyéthers oxygénés, soufrés, cycliques ou macrocycliques.

2. Procédé selon la revendication 1, caractérisé en ce que le carbonate de tertioalcoyle et d'alcalin est un sel de sodium.

3. Procédé selon la revendication 1, caractérisé en ce que l'halogénure d'acide est le chlorure trichloracétyle ou de dichloroacétyle.

4. Procédé selon la revendication 1, caractérisé en ce que la réaction est réalisée dans un solvant aromatique, de préférence dans le toluène.

5. Procédé selon la revendication 1, caractérisé en ce que les amines tertiaires oxygénées répondent à la formule :

$$N\left[CHR_1 - CHR_2 - O - (CHR_3 - CHR_4 - O)_n - R_5\right]_3$$

dans laquelle n est un nombre entier supérieur ou égal à 0 et inférieur ou égal à 10 environ ($0 \leqq n \leqq 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $-C_mH_{2m}-ø$, ou $C_mH_{2m+1}-ø$, m étant compris entre 1 et environ 12.

6. Procédé selon la revendication 1 caractérisé en ce que les agents complexants répondent aux formules II suivantes :

$$R_{10} - Z \left[A - D\right]_p A - Z - R_{10}$$

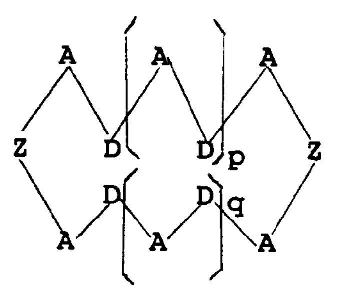

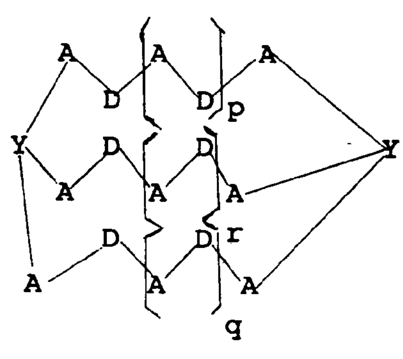

dans lesquelles

- Z représente O ou $Y-R_{10}$
- Y représente N ou P

- A représente un groupement alkylène ayant de 1 à 3 atomes de carbone.
- D représente O, S ou N-$R_{11}$ où $R_{11}$ représente un radical alkyle ayant de 1 à 6 atomes de carbone.
- $R_{10}$ peut représenter un radical alkyle ayant 1 à 6 atomes de carbone, p, q et r identiques ou différents sont des nombres entiers compris entre 1 et 5.

7. Procédé selon la revendication 5 caractérisé en ce que l'amine oxygénée est la trisdioxaheptylamine.

8. Procédé selon les revendications 1 et 6 caractérisé en ce que le polyéther est le diéther méthylique du diéthylène glycol.

9. Procédé selon la revendication 1 caractérisé en ce que le polyéther est le dioxanne.

## Patentansprüche

1. Verfahren zur Herstellung von Di-tert.-alkyldicarbonaten, dadurch gekennzeichnet, daß man ein tert.-Alkalialkylcarbonat mit einem Säurehalogenid in Gegenwart eines Komplexierungsmittels aus der Gruppe von Sauerstoff enthaltenden tert. Aminen, Sauerstoff oder Schwefel enthaltenden cyclischen oder makrocyclischen Polyethern reagieren läßt.

2. Verfahren nach Anspruch 1, dadurch gekennzeichnet, daß das tert.-Alkalialkylcarbonat ein Natriumsalz ist.

3. Verfahren nach Anspruch 1, dadurch gekennzeichnet, daß das Säurehalogenid Tri- oder Dichloracetylchlorid ist.

4. Verfahren nach Anspruch 1, dadurch gekennzeichnet, daß die Reaktion in einem aromatischen Lösemittel, vorzugsweise in Toluol durchgeführt wird.

5. Verfahren nach Anspruch 1, dadurch gekennzeichnet, daß die Sauerstoff enthaltenden tert. Amine der Formel

$$N\left[CHR_1-CHR_2-O-(CHR_3-CHR_4-O)_n-R_5\right]_3$$

entsprechen, in der n eine ganze Zahl zwischen 0 und ungefähr 10 ($0 \leqq n \leqq 10$) ist, $R_1$, $R_2$, $R_3$, $R_4$, gleich oder verschieden, ein Wasserstoffatom oder ein Alkylrest mit 1 bis 4 Kohlenstoffatomen und $R_5$ einen Alkyl- oder Cycloalkylrest mit 1 bis 12 Kohlenstoffatomen, einen Phenylrest oder einen Rest der Formel $-C_mH_{2m}-ø$ oder $-C_mH_{2m+1}-ø$, mit m zwischen 1 und etwa 12 bedeuten.

6. Verfahren nach Anspruch 1, dadurch gekennzeichnet, daß die Komplexierungsmittel den folgenden Formeln II entsprechen

$$R_{10}-Z-\{A-D\}_p-A-Z-R_{10}$$

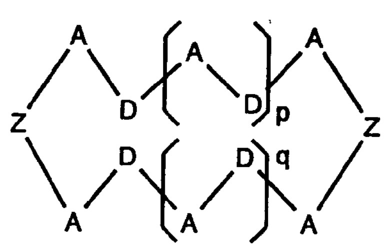

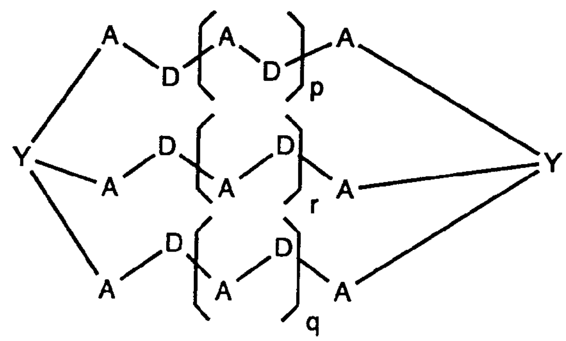

in denen

- Z O oder Y-$R_{10}$ darstellt
- Y N oder P darstellt
- A eine Alkylengruppe mit 1 bis 3 Kohlenstoffatomen darstellt
- D O, S oder N-$R_{11}$ darstellt, wobei $R_{11}$ ein Alkylrest mit 1 bis 6 Kohlenstoffatomen ist.
- $R_{10}$ kann einen Alkylrest mit 1 bis 6 Kohlenstoffatomen darstellen; p, q, und r, gleich oder verschieden, sind ganze Zahlen zwischen 1 und 5.

**7.** Verfahren nach Anspruch 5, dadurch gekennzeichnet, daß das Sauerstoff enthaltende Amin Tris-dioxaheptylamin ist.

**8.** Verfahren nach Anspruch 1 und 6, dadurch gekennzeichnet, daß der Polyether Diethylenglycoldimethylether ist.

**9.** Verfahren nach Anspruch 1, dadurch gekennzeichnet, daß der Polyether Dioxan ist.

## Claims

**1.** Process for the preparation of di-tert, alkyl dicarbonate, characterized in that contacting takes place between an alkali metal and a tert. alkyl carbonate and an acid halide in the presence of a complexing agent chosen from among oxygenated tertiary amines and oxygenated, sulphurized, cyclic or macrocyclic polyethers.

**2.** Process according to claim 1, characterized in that the alkali metal and tert. alkyl carbonate is a sodium salt.

**3.** Process according to claim 1, characterized in that the acid halide is trichloroacetyl or dichloroacetyl chloride.

**4.** Process according to claim 1, characterized in that the reaction is carried out in an aromatic solvent, preferably in toluene.

**5.** Process according to claim 1, characterized in that the oxygenated tertiary amines comply with the formula:

$$N \left[ CHR_1 - CHR_2 - O - (CHR_3 - CHR_4 - O)_n - R_5 \right]_3$$

in which n is an integer equal to or greater than 0 and equal to or below approximately 10($0 \leqq n \leqq 10$), $R_1$, $R_2$, $R_3$ and $R_4$, which can be the same or different, represent a hydrogen atom or an alkyl radical with 1 to 4 carbon atoms and $R_5$ represents an alkyl or cycloalkyl radical with 1 to 12 carbon atoms, a phenyl radical or a radical of formula $-C_m H_{2m} - \phi$ or $C_m H_{2m+1} - \phi$, m being between 1 and approximately 12.

**6.** Process according to claim 1, characterized in that the complexing agents comply with the following formulas (11):

$$R_{10} - Z \{A - D\}_p A - Z - R_{10}$$

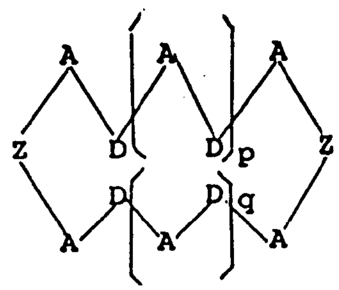

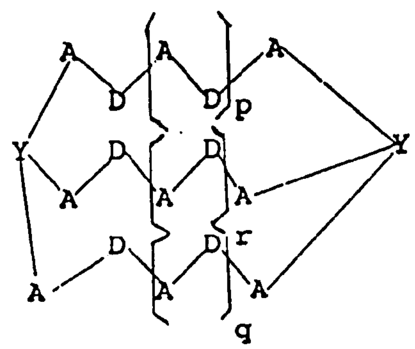

in which:

Z represents 0 or Y-$R_{10}$,

Y represents N or P,

A represents an alkylene group having 1 to 3 carbon atoms,

D represents O, S or N-$R_{11}$, in which $R_{11}$ represents an alkyl radical having 1 to 6 carbon atoms,

$R_{10}$ can represent an alkyl radical having 1 to 6 carbon atoms, whilst p, q and r, which can be the same or different are integers between 1 and 5.

**7.** Process according to claim 5, characterized in that the oxygenated amine is tris dioxaheptyl amine.

**8.** Process according to claims 1 and 6, characterized in that the polyether is diethylene glycol methyl diether.

**9.** Process according to claim 1, characterized in that the polyether is dioxan.